# EUROPÄISCHE PATENTSCHRIFT

(11) **EP 1 969 132 B1**
(45) Veröffentlichungstag und Bekanntmachung des Hinweises auf die Patenterteilung: **02.03.2011**
(21) Anmeldenummer: 06830606.7
(22) Anmeldetag: 13.12.2006
(51) Int. Cl.: C12P 17/16, C07D 333/20, C07D 333/16

(54) **ENZYMATISCHE REDUKTION ZUR HERSTELLUNG OPTISCH AKTIVER ALKOHOLE**
ENZYMATIC REDUCTION FOR PRODUCING OPTICALLY ACTIVE ALCOHOLS
REDUCTION ENZYMATIQUE POUR PRODUIRE DES ALCOOLS OPTIQUEMENT ACTIFS

(30) Priorität: 23.12.2005 DE 102005062662
(43) Veröffentlichungstag der Anmeldung: 17.09.2008
(73) Patentinhaber: BASF SE, 67056 Ludwigshafen (DE)
(72) Erfinder: STÜRMER, Rainer, 67127 Rödersheim-Gronau (DE); KESSELER, Maria, 68239 Mannheim (DE); HAUER, Bernhard, 67136 Fussgönheim (DE); FRIEDRICH, Thomas, 64283 Darmstadt (DE); BREUER, Michael, 64285 Darmstadt (DE); SCHRÖDER, Hartwig, 69226 Nussloch (DE)
(74) Vertreter: Popp, Andreas
(86) Internationale Anmeldenummer: PCT/EP2006/069680
(87) Internationale Veröffentlichungsnummer: WO 2007/074070

(56) Entgegenhaltungen:
- WO-A-2004/090094
- WO-A-2005/033094
- WO-A-2005/108590
- ITOH N ET AL: "Chiral alcohol production by NADH-dependent phenylacetaldehyde reductase coupled with in situ regeneration of NADH" EUROPEAN JOURNAL OF BIOCHEMISTRY, BERLIN, DE, Bd. 269, Nr. 9, Mai 2002 (2002-05), Seiten 2394-2402, XP002221911 ISSN: 0014-2956

## Beschreibung

Die vorliegende Erfindung betrifft ein Verfahren zur Herstellung von optisch aktiven Alkanolen durch enzymatische Reduktion der entsprechenden Ketone, insbesondere die Herstellung von (1S)-3-Methylamino-1-(2-thienyl)-propan-1-ol und (1S)-3-Chlor-1-(2-thienyl)-propan-1-ol.

### Stand der Technik:

(1S)-3-Methylamino-1-(2-thienyl)-propan-1-ol ("Duloxetinealkohol") ist Baustein in der Duloxetine-Synthese. Duloxetine® ist ein Pharmawirkstoff, der sich momentan in der Zulassung befindet und im Indikationsgebiet Depression und Inkontinenz eingesetzt werden soll.

EP-B-0273658 beschreibt ein Verfahren zur Herstellung der korrespondierenden Base von Duloxetine durch Umsetzung von 2-Acetylthiophen in einer Mannich-Reaktion mit Formaldehyd und Dimethylamin, Reduktion der Ketogruppe der dabei erhaltenen Mannich-Base zum racemischen (S)-3-N,N-Dimethylamino-1-(thien-2-yl)propan-1-ol, Veretherung der Alkoholfunktion mit Naphthylfluorid und schließlich Umwandlung der Dimethylamino-Gruppe in eine Methylamino-Funktion. Das gewünschte Enantiomer des Naphtylethers erhält man durch Einsatz chiraler Ausgangsmaterialien oder durch Racemattrennung auf der Stufe des Endprodukts, beispielsweise über die Salze mit optisch aktiven Säuren oder Chromatographie an einer chiralen stationären Phase.

US-5,362,886 beschreibt ein analoges Verfahren, bei dem das nach Reduktion der Ketogruppe erhaltene racemische Propanol mit S-Mandelsäure versetzt wird. Das hierbei erhaltene S-Enantiomer des Alkohols wird in die nachfolgenden Reaktionsstufen eingesetzt.
EP-A-0457559 beschreibt ebenfalls ein der EP-B-0273658 analoges Verfahren. Hierbei wird die Ketogruppe der Mannich-Base mit dem asymmetrischen Reduktionssystem LAH-lcb (Lithiumaluminiumhydrid-[(2R,2S)-(-)-4-Dimethylamino-1,2-diphenyl-3-methyl-2-butanol]) zum Alkohol in Form des S-Enantiomers reduziert. Nachteilig hierbei ist neben den Kosten die Empfindlichkeit des Reduktionssystems LAH-lcb, das nur wenige Minuten stabil ist.

W. J. Wheeler und F. Kuo beschreiben in Journal of Labelled Compounds and Radiopharmaceuticals, Band XXXVI, Nr. 3, Seite 213 bis 223 ein Verfahren zur Herstellung von Duloxetine. Hierzu wird Thiophen-2-carbonsäurechlorid in einer Stille-Kopplung mit Vinyl-tri-n-butylstannan in Gegenwart katalytischer Mengen Benzylchlor-bis(triphenylphosphin)palladium(II) in DMPU (Dimethylpropylenharnstoff) zu 1-(Thien-2-yl)-propenon der Formel (V) 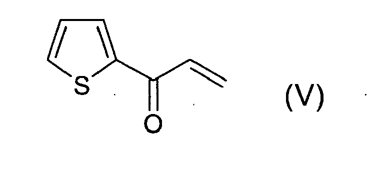 umgesetzt, welches anschließend durch Behandlung mit Chlorwasserstoff in 3-Chlor-1-(thien-2-yl)-propan-1-on der Formel (VI) 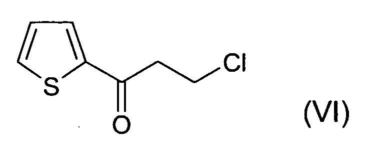 überführt wird. Das so erhaltene Chlorpropanon wird anschließend unter Verwendung eines chiralen Oxazaborylidins und BH₃ zu (*S*)-3-Chlor-1-(thien-2-yl)-propan-1-ol der Formel (VII) 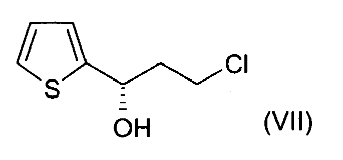 ()
reduziert. Der so erhaltene Alkohol wird durch sukzessive Umsetzung mit Natriumiodid und anschließend mit Methylamin in (*S*)-3-Methylamino-1-(thien-2-yl)-propan-1-ol überführt. Durch nachfolgende sukzessive Umsetzung mit Natriumhydrid, 1-Fluornaphthalin und Chlorwasserstoff erhält man Duloxetine in Form des Hydrochlorids. 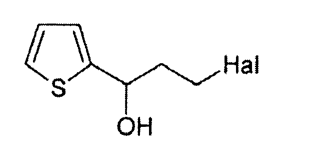 Hal = Halogen

WO 05/108590 beschreibt Dehydrogenasen zur Herstellung von optisch aktiven Alkanolen durch Reduktion der entsprechenden Alkanone, insbesondere der Herstellung von 3-Methylamino-1-(2-thienyl)-propanon und 3-Chlor-1-(2-thienyl)-propanon.

WO 05/033094 beschreibt enzymatische und nicht-enzymatische Verfahren zur Herstellung von 3-Methylamino-1-(thien-2-yl)-propan-1-ol sowie Enzyme zur Durchführung dieser Verfahen.

### Kurze Beschreibung der Erfindung:

Der Erfindung lag daher die Aufgabe zugrunde, eine Weg zur stereospezifischen Reduktion von substituierten Alkanonen, wie dem 3-Methylamino-1-(2-thienyl)-propanon und dem 3-Chlor-1-(2-thienyl)-propanon, zu finden.

Diese Aufgabe wurde durch den überraschenden Befund gelöst, dass Enzyme mit Dehydrogenase-Aktivität, die aus Mikroorganismen der Gattung Lactobacillus, insbesondere der Art Lactobacillus brevis herstellbar sind, zur stereospezifischen Katalyse der obigen Reaktion befähigt sind.

Ein erster Gegenstand der Erfindung betrifft ein Verfahren zur Herstellung von optisch aktiven Alkanolen der Formel I 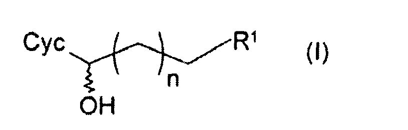 worin
- n: für einen ganzzahligen Wert von 0 bis 5 steht;
- Cyc: für einen gegebenenfalls substituierten, ein- oder mehrkernigen, gesättigten oder ungesättigten, carbocyclischen oder heterocyclischen Ring steht, und
- R¹: für Halogen, SH, OH, NO₂, NR²R³ oder NR²R³R⁴⁺X⁻ steht, wobei R², R³ und R⁴ unabhängig voneinander für H oder einen Niedrig(C₁-C₆)alkyl- oder Niedrig(C₁-C₆)alkoxy-Rest stehen und X⁻ für ein Gegenion steht,
wobei man in einem Alkanon der Formel II 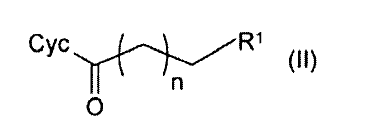 worin n, Cyc und R¹ die oben angegebenen Bedeutungen besitzen, enthaltenden Medium, ein Enzym mit einer Polypeptidsequenz
(i) SEQ ID NO: 1oder
(ii) bei der bis zu 25% der Aminosäurereste gegenüber SEQ ID NO:1 durch Deletion, Insertion, Substitution oder einer Kombination davon verändert sind und die noch mindestens 50% der enzymatischen Aktivität von SEQ ID NO:1 besitzt, inkubiert,
wobei die Verbindung der Formel II zur Verbindung der Formel I enzymatisch reduziert wird, und man das gebildete Produkt isoliert.
In einer besonders bevorzugten Ausführungsform dient das Verfahren zur Herstellung von Derivaten des 1-(2-thienyl)-(S)-propanol der Formel III 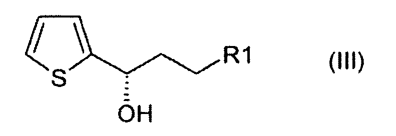 wobei R¹ = Cl oder NHCH₃ bedeuten,
wobei man in einem ein Derivat des 1-(2-thienyl)-propanons der Formel IV 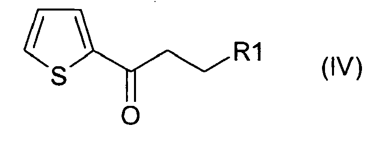 enthaltenden Medium diese Verbindung zur Verbindung der Formel III enzymatisch reduziert wird, und man das in im Wesentlichen enantiomerenreiner Form gebildete Produkt isoliert.

Vorzugsweise verwendet man bei diesen Verfahren ein Enzym mit Dehydrogenase-Aktivität, das aus Mikroorganismen der Gattungen Lactobacillus herstellbar ist.

Besonders bevorzugt verwendet man Dehydrogenasen aus Lactobacillus brevis.

In einer besonders bevorzugten Ausführungsform des Verfahrens ist das Enzym mit Dehydrogenase-Aktivität ausgewählt unter Enzymen, welche eine Aminosäuresequenz gemäß SEQ ID NO: 1 umfassen oder eine davon abgeleitete Sequenz, bei der bis zu 25%, bevorzugt bis zu 20%, besonders bevorzugt bis zu 15% insbesondere bis zu 10,9,8,7,6,5,4,3,2,1 % der Aminosäurereste durch eine Deletion, eine Substitution eine Insertion oder eine Kombination aus Deletion, Substitution und Insertion verändert worden sind, wobei die gegenüber SEQ ID NO: 1 veränderten Polypeptidsequenzen noch mindestens 50%, bevorzugt 65%, besonders bevorzugt 80% , insbesondere mehr als 90 % der enzymatischen Aktivität von SEQ ID NO:1 besitzten. In diesem Zusammenhang soll unter enzymatische Aktivität von SEQ ID NO:1 die Fähigkeit verstanden werden, die Ketone der Formel (IV) mit R¹ = Cl enantioselektiv zu dem (S)-Alkohol mit der allgemeinen Formel (III) zu reduzieren.

Vorzugsweise wird das erfindungsgemäße Verfahren unter Zugabe von Reduktionsäquivalenten (NADH oder NADPH) oder die bei der Umsetzung verbrauchten Reduktionsäquivalente regenerierenden (biochemischen oder elektrochemischen) Bedingungen durchgeführt. Für diese bevorzugte Ausführungsformen wird auf die Beispiele 5 und 6 verwiesen, die allgemein anwendbare Regenerationssysteme beschreiben.

Eine weitere geeignete Ausführunsform für eine Cofaktor-Regenerierung bei dem erfindungsgemässen Verfahren ist die Verwendung eines nicht mit Wasser mischbaren oxidierbaren Verbindung, beispielsweise eines höheren Alkohols, bevorzugt Hexanol, der in einer zweiten flüssigen Phase im Reaktionsmedium vorliegen kann.

Weiterhin ist es bevorzugt, die Umsetzung der Verbindung der allgemeinen Formel II, wie z.B. der Formel IV, in Gegenwart eines Mikroorganismus erfolgen zu lassen, der ausgewählt ist unter Bakterien der Familien Enterobacteriaceae, Pseudomonadaceae, Rhizobiaceae, Streptomycetaceae und Nocardiaceae. Der Mikroorganismus kann insbesondere ein rekombinanter Mikroorganismus sein, der mit einem Nukleinsäurekonstrukt transformiert ist, welche für ein Enzym mit Dehydrogenase-Aktivität gemäß obiger Definition kodiert.

Gegenstand der Erfindung ist insbesondere
- einen ein Enzym mit Dehydrogenase-Aktivität produzierenden Mikroorganismus aus einer natürlichen Quelle isoliert oder rekombinant herstellt,
- diesen Mikroorganismus vermehrt,
- aus dem Mikroorganismus das Enzym mit Dehydrogenase-Aktivität gegebenenfalls isoliert oder eine dieses Enzym enthaltende Proteinfraktion herstellt, und
- den Mikroorganismus gemäß Stufe b) oder das Enzym gemäß Stufe c) in ein Medium überführt, das eine Verbindung der Formel I enthält.

Gegenstand der Erfindung sind Enzyme mit Dehydrogenase Aktivität mit einer Polypeptidsequenz
(i) SEQ ID NO: 1 oder
(ii) bei der bis zu 25% der Aminosäurereste gegenüber SEQ ID NO:1 durch Deletion, Insertion, Substitution oder einer Kombination davon verändert sind und die noch mindestens 50% der enzymatischen Aktivität von SEQ ID NO:1 besitzt.

Gegenstand der Erfindung sind außerdem kodierende Nukleinsäuresequenzen, umfassend die kodierende Sequenz für ein Polypeptid gemäß obiger Definition.

Weiterhin betrifft die Erfindung Expressionskassetten, umfassend in operativer Verknüpfung mit wenigstens einer regulativen Nukleinsäuresequenz eine kodierende Nukleinsäuresequenz gemäß obiger Definition.

Ein weiterer Gegenstand der Erfindung sind rekombinante Vektoren, umfassend wenigstens eine solche Expressionskassette.

Die Erfindung betrifft auch prokaryotische oder eukaryotische Wirte, welche mit wenigstens einem erfindungsgemäßen Vektor transformiert sind.

Ein letzter Gegenstand der Erfindung betrifft die Verwendung eines Enzyms mit Dehydrogenase-Aktivität gemäß obiger Definition oder eines dieses Enzym produzierenden Mikroorganismus zur Herstellung von Verbindungen der Formeln I oder III, und deren Weiterverarbeitung beispielsweise zur Herstellung von Duloxetine (Formel VIII)

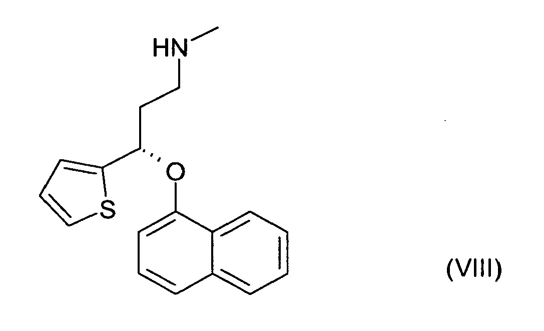

### Detaillierte Beschreibung der Erfindung:

### A. Allgemeine Begriffe und Definitionen

Werden keine andere Angaben gemacht, so gelten folgende allgemeine Bedeutungen:
"Halogen" steht für Fluor, Chlor, Brom oder Jod , insbesondere Fluor oder Chlor.
"Niedrigalkyl" steht für geradkettige oder verzweigte Alkylreste 1 bis 6 C-Atomen, wie Methyl, Ethyl, i- oder n-Propyl, n-, i-, sec.- oder tert.-Butyl, n-Pentyl oder 2-MethylButyl, n-Hexyl, 2-Methyl-pentyl, 3-Methyl-pently, 2-Ethyl-butyl.
"Niedrigalkenyl" steht für die ein- oder mehrfach, vorzugsweise einfach oder zweifach ungesättigten Analoga oben genannter Alkylreste mit 2 bis 6 Kohlenstoffatomen, wobei die Doppelbindung in beliebiger Position der Kohlenstoffkette liegen kann.
"Niedrigalkoxy" steht für die Sauerstoff-terminierten Analoga obiger Alkylreste.
"Aryl" steht für einen ein- oder mehrkernigen, vorzugsweise ein- oder zweikernigen, gegebenenfalls substituierten aromatischen Rest, insbesondere für Phenyl oder für ein über eine beliebige Ringposition gebundenes Naphthyl, wie 1- oder 2-Naphthyl. Diese Arylreste können gegebenenfalls 1 oder 2 gleiche oder verschiedene Substituenten tragen, ausgewählt unter Halogen, Niedrigalkyl, Niedrigalkoxy gemäß obiger Definition oder Trifluormethyl.

### Substituierte Alkanone, (S)-Alkanole und Derivate davon

Erfindungsgemäß durch enzymatische Katalyse zugängliche Alkanole sind solche obiger Formel (I) worin
- n: für einen ganzzahligen Wert von 0 bis 5 steht;
- Cyc: für einen gegebenenfalls substituierten, ein- oder mehrkernigen, gesättigten oder ungesättigten, carbocyclischen oder heterocyclischen Ring steht, und
- R¹: für Halogen, SH, OH, NO₂, NR²R³ oder NRZR³R⁴⁺X⁻ steht, wobei R², R³ und R⁴ unabhängig voneinander für H oder einen Niedrigalkyl- oder Niedrigalkoxy-Rest stehen und X- für ein Gegenion steht.

Die zur enzymatischen Synthese verwendeten Alkanone obiger Formel II sind an sich bekannte Verbindungen und unter Anwendung allgemein bekannter organischer Syntheseverfahren zugänglich (vgl. z.B. EP-A- 0 273 658).

Vorzugsweise steht n in obigen Verbindungen für 0, 1 oder 2, insbesondere für 1.

Als Beispiele für carbo- und heterocyclische Gruppen Cyc sind insbesondere ein- oder zweikernigen, vorzugsweise einkernige, Gruppen mit bis zu 4, vorzugsweise 1 oder 2 gleichen oder verschiedenen Ring-Heteroatomen, ausgewählt unter O, N und S sind zu nennen:

Diese carbo- oder heterocyclischen Ringe umfassen insbesondere 3 bis 12, vorzugsweise 4, 5 oder 6 Ring-Kohlenstoffatomen. Als Beispiele können genannt werden Cyclopropyl, Cyclobutyl, Cyclopenty, Cyclohexyl, Cycloheptyl, die ein- oder mehrfach ungesättigten Analoga davon, wie Cyclobutenyl, Cyclopentenyl, Cyclohexenyl, Cycloheptenyl, Cyclohexadienyl, Cycloheptadienyl; sowie 5- bis 7-gliedrige gesättigte oder ein oder mehrfach ungesättigte heterocyclische Reste mit 1 bis 4 Heteroatomen, die ausgewählt sind unter O, N und S, wobei der Heterocyclus gegebenenfalls mit einem weiteren Heterocyclus oder Carbocyclus kondensiert sein kann. Insbesondere sind zu nennen heterocyclische Reste, abgeleitet von Pyrrolidin, Tetrahydrofuran, Piperidin, Morpholin, Pyrrol, Furan, Thiophen, Pyrazol, Imidazol, Oxazol, Thiazol, Pyridin, Pyran, Pyrimidin, Pyridazin, Pyrazin, Cumaron, Indol und Chinolin.

Die Reste Cyc können dabei über eine beliebige Ringposition, vorzugsweise über ein Ring-Kohlenstoffatom, an das Alkanon bzw. das Alkanol gebunden sein.

Beispiele für geeignet Cyc-Reste sind 2-Thienyl, 3-Thienyl; 2-Furanyl, 3-Furanyl; 2-Pyridyl, 3-Pyridyl oder 4-Pyridyl; 2-Thiazolyl, 4-Thiazolyl oder 5-Thiazolyl; 4-Methyl-2-thienyl, 3-Ethyl-2-thienyl, 2-Methyl-3-thienyl, 4-Propyl-3-thienyl, 5-n-Butyl-2-thienyl, 4-Methyl-3-thienyl, 3-Methyl-2-thienyl; 3-Chlor-2-thienyl, 4-Brom-3-thienyl, 2-lod-3-thienyl, 5-lod-3-thienyl, 4-Fluor-2-thienyl, 2-Brom-3-thienyl, und 4-Chlor-2-thienyl.

Die Reste Cyc können weiterhin ein- oder mehrfach, wie z.B. ein- oder zweifach, substituiert sein. Vorzugsweise sitzen die Substituenten an einem Ring-Kohlenstoffatom. Beispiele für geeignete Substituenten sind Halogen, Niedrigalkyl, Niedrigalkenyl, Niedrigalkoxy, -OH, -SH, -NO₂ oder NR²R³, wobei R² und R³ obige Bedeutungen besitzen, bevorzugt Halogen oder Niedrigalkyl.

R¹ steht insbesondere für Halogen, NR²R³ oder NR²R³R⁴⁺X⁻, wobei R², R³ bzw. R², R³ und R⁴ unabhängig voneinander für H oder einen Niedrigalkyl- oder Niedrigalkoxy-Rest stehen und X- für ein Gegenion steht, wobei vorzugsweise einer der Reste R², R³ und R⁴ für H steht. Geeignete Gegenionen sind beispielsweise Säureanionen, wie sie beispielsweise bei Herstellung eines Säureadditionssalzes anfallen. Beispiel hierzu sind z.B. in der EP-A-0 273 658 genannt, worauf hiermit Bezug genommen wird. Bevorzugte Beispiele für Reste R¹ sind insbesondere Fluor oder Chlor, sowie NR²R³ worin R² und R³ gleich oder verschieden sind und für H oder Methyl, Ethyl oder n-Propyl stehen; besonders bevorzugt steht R¹ für Chlor oder-NHMethyl.

### C. Geeignete Enzyme mit Dehydrogenase-Aktivität

Bevorzugte Enzyme mit Dehydrogenase-Aktivität umfassen eine Aminosäuresequenz gemäß SEQ ID NO: 1.

Erfindungsgemäß mit umfasst sind ebenfalls "funktionale Äquivalente" der konkret offenbarten Enzyme mit Dehydrogenase-Aktivität und die Verwendung dieser in den erfindungsgemäßen Verfahren.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die von 3-Chlor-1-(thien-2-yl)-propan-1-on zum entsprechenden S-Alkohol reduzieren und die mindestens 50 %, bevorzugt 60 %, besonders bevorzugt 75 %, ganz besonders bevorzugt 90 % der Aktivität eines Enzyms mit der in SEQ ID NO:1 aufgeführten Aminosäuresequenz aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktiviät.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäüre und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90%, 95% oder 99%, Homologie zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Im. Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation oder Verkürzung des Proteins.

Homologe des erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtiicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; lke et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### D. Für die Dehydrogenasen kodierende Nukleinsäuresequenzen

Gegenstand der Erfindung sind insbesondere Nukleinsäuresequenzen (einzel- und doppelsträngige DNA- und RNA-Sequenzen, wie z.B. cDNA und mRNA), die für ein Enzym mit erfindungsgemäßer Dehydrogenase-Aktivität kodieren. Bevorzugt sind Nukleinsäuresequenzen, welche z.B. für Aminosäuresequenzen gemäß SEQ ID NO:1 oder charakteristische Teilsequenzen davon kodieren. Die entsprechenden Nukleinsäuren lassen sich leicht durch Rückübersetzung der SEQ ID NO:1 gemäß dem genetischen Code ermitteln. Bevorzugt ist auch die Adaption der Nukleinsäuresequenz an die "codon usage" des geplanten Wirtsorganismus, in dem die Nukleinsäure exprimiert werden soll.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Die erfindungsgemäßen Nukleinsäuresequenzen lassen sich prinzipiell aus allen Organismen der Gattung Lactobacillus, insbesondere aus Lactobacillus brevis identifizieren und isolieren. Erfindungsgemäße Nukleinsäuresequenzen lassen sich beispielsweise mit üblichen Hybridisierungsverfahren oder der PCR-Technik aus anderen Organismen, z.B. über genomische oder cDNA-Banken, isolieren. Diese DNA-Sequenzen hybridisieren unter Standardbedingungen mit den erfindungsgemäßen Sequenzen. Zur Hybridisierung werden vorteilhaft kurze Oligonukleotide der konservierten Bereiche beispielsweise aus dem aktiven Zentrum, die über Vergleiche mit einer erfindungsgemäßen Dehydrogenase in dem Fachmann bekannter Weise ermittelt werden können, verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Gegenstand der Erfindung sind auch Derivate der konkret offenbarten oder ableitbaren Nukleinsäuresequenzen.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten einer erfindungsgemäßen Nukleinsäuresequenz sind beispielsweise Allelvarianten zu verstehen, die mindestens 40 % Homologie auf der abgeleiteten Aminosäureebene, bevorzugt mindestens 60 % Homologie, ganz besonders bevorzugt mindestens 80, 85, 90, 93, 95 oder 98 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen beispielsweise pilzliche oder bakterielle Homologe, verkürzte Sequenzen, Einzelstrang-DNA oder RNA der kodierenden und nichtkodierenden DNA-Sequenz, zu verstehen. So besitzten z.B. auf DNA-Ebene eine Homologie von mindestens 40 %, bevorzugt von mindestens 60 %, besonders bevorzugt von mindestens 70 %, ganz besonders bevorzugt von mindestens 80 % über den gesamten angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -1000 Basen stromaufwärts vor dem Startkodon oder 0 bis 1000 Basen stromabwärts nach dem Stopkodon so verändert wurden, dass die Genexpression und/oder die Proteinexpression verändert, bevorzugt erhöht wird.

Weiterhin umfasst die Erfindung auch Nukleinsäuresequenzen, welchen mit oben genannten kodierenden Sequenzen unter "stringenten Bedingungen" hybridisieren. Diese Polynukleotide lassen sich bei der Durchmusterung von genomischen oder cDNA-Banken auffinden und gegebenenfalls daraus mit geeigneten Primern mittels PCR vermehren und anschließend beispielsweise mit geeigneten Sonden isolieren. Darüber hinaus können erfindungsgemäße Polynukleotide auch auf chemischem Wege synthetisiert werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

### E. Ausgestaltungen erfindungsgemäßer Konstrukte

Gegenstand der Erfindung sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Unter einem erfindungsgemäßen Nukleinsäurekonstrukt sind insbesondere solche zu verstehen, bei weichen das Gen für eine erfindungsgemäße Dehydrogenase mit einem oder mehreren Regulationssignalen zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das erfindungsgemäße Verfahren sind beispielsweise in Promotoren wie cos-, tac-, trp-, tet-, trp-tet-, lpp-, lac-, lpp-lac-, lacl^{q-,} T7- , T5-, T3-, gal-, trc-, ara-, rhaP (rhaP_{BAD})SP6-, lambda-P_{R}- oder im lambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. In diesem Zusammenhang sind auch die Promotoren der Pyruvatdecarboxylase und der Methanoloxidase, beispielsweiseaus Hansenula vorteilhaft. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar. Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, Igt11 oder pBdCl, in Streptomyces pIJ101, plJ364, plJ702 oder plJ361, in Bacillus pUB110, pC194 oder pBD214, in Corynebacterium pSA77 oder pAJ667, in Pilzen pALS1, plL2 oder pBB116, in Hefen 2alphaM, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### F. Erfindungsgemäß brauchbare Wirtsorganismen

Mit Hilfe der erfindungsgemäßen Vektoren oder Konstrukte sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Erfindungsgemäß sind auch homolog rekombinierte Mikroorganismen herstellbar. Dazu wird ein Vektor hergestellt, der zumindest einen Abschnitt eines erfindungsgemäßen Gens oder einer kodierenden Sequenz enthält, worin gegebenenfalls wenigstens eine Aminosäure-Deletion, -Addition oder -Substitution eingebracht worden ist, um die erfindungsgemäße Sequenz zu verändern, z.B. funktionell zu disrumpieren ("Knockout"-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, daß das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des erfindungsgemäßen Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit erfindungsgemäßer Dehydrogenaseaktivität kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

### G. Rekombinante Herstellung der erfindungsgemäßer Polypeptide

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### H. Durchführung des erfindungsgemäßen Verfahrens zur Herstellung von (S)-Alkanolen

Die erfindungsgemäss verwendeten Enzyme mit Dehydrogenase-Aktivität können im erfindungsgemäßen Verfahren als freies oder immobilisiertes Enzym verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten bzw. optisch aktiven Alkoholen sind im erfindungsgemäßen Verfahren Enantiomere zu verstehen, die eine Enantiomerenanreicherung zeigen. Bevorzugt werden im Verfahren Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Werden für das erfindungsgemäße Verfahren freie Organismen oder Enzyme verwendet, so werden diese vor der Extraktion zweckmäßigerweise abgetrennt beispielsweise über eine Filtration oder Zentrifugation.

Das im erfindungsgemäßen Verfahren hergestellte Produkt, beispielsweise (1S)-3-Methylamino-1-(2-thienyl)-propan-1-ol, lässt sich vorteilhaft aus der wässrigen Reaktionslösung über Extraktion oder Destillation gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetat, Diisopropylether, Benzol, MTBE oder Essigester, ohne darauf beschränkt zu sein.

Nach Einengen der organischen Phase können die Produkte in der Regel in guten chemischen Reinheiten, das heißt größer 80 % chemische Reinheit, gewonnen werden. Nach Extraktion kann die organische Phase mit dem Produkt aber auch nur zum Teil eingeengt werden und das Produkt auskristallisiert werden. Dazu wird die Lösung vorteilhaft auf eine Temperatur von 0 °C bis 10 °C abgekühlt. Die Kristallisation kann auch direkt aus der organischen Lösung oder aus einer wässrigen Lösung erfolgen. Das auskristallisierte Produkt kann nochmals im gleichen oder in einem anderen Lösungsmittel zur erneuten Kristallisation aufgenommen werden und nochmals kristallisiert werden. Durch die anschließende vorteilhafte mindestens einmalig durchgeführte Kristallisation kann die Enantiomerenreinheit des Produktes falls erforderlich weiter gesteigert werden.

Bei den genannten Aufarbeitungsarten lässt sich das Produkt des erfindungsgemäßen Verfahrens in Ausbeuten von 60 bis 100 %, bevorzugt von 80 bis 100 %, besonders bevorzugt von 90 bis 100 %, bezogen auf das für die Reaktion eingesetzte Substrat, wie z.B. von 3-Methylamino-1-(2-thienyl)-propan-1-on, isolieren. Das isolierte Produkt zeichnet sich durch eine hohe chemische Reinheit von > 90 %, bevorzugt > 95 % besonders bevorzugt von > 98 % aus. Weiterhin haben die Produkt eine hohe Enantiomerenreinheit, die vorteilhaft falls erforderlich durch die Kristallisation weiter gesteigert werden kann.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die obige Beschreibung und die nachstehenden Beispiele dienen nur der Verdeutlichung der Erfindung. Die für den Fachmann offensichtlichen, zahlreich möglichen Abwandlungen sind erfindungsgemäß ebenfalls umfasst.

### Experimenteller Teil:

### Beispiel 1

### Analytik von 3-Chlor-1-(thien-2-yl)-propan-1-on und 3-Chlor-1-(thien-2-yl)-propan-1-ol

Die Konzentration von 3-Chlor-1-(thien-2-yl)-propan-1-on und 3-Chlor-1-(thien-2-yl)-propan-1-ol lassen sich mittels HPLC bestimmen. Je nach Wahl der stationären und mobilen Phase lassen sich neben der Konzentration auch ee-Wert bestimmen.

### a) achirale Analytik

Die Quantifizierung der Umsetzung wurde mit folgendem System durchgeführt:
- stationäre Phase:: Chromoltih SpeedROD RP18, 50*4, 6µm, Merck (Darmstadt) tem- periert auf 45°C
- mobilePhase:: Laufmittel A: 10mM KH₂PO₄, pH 2.5 Laufmittel B: Acetonitril Gradient: 0-0.5 min, 35%B; 0.5-1.0 min 35 auf 80%B; 1.0-1.2 min 80%B; 1.2-1.3 min 80% - 35%B; 1.3-2.0 min 35%B;
- Flussrate:: 1.5 ml/min
- Detektion:: UV-Detektion bei 230 und 260nm
- Retentionszeiten:: 3-Chlor-1-(thien-2-yl)-propan-1-on: ca. 1.6 min 3-Chlor-1-(thien-2-yl)-propan-1-ol: ca. 1.3 min

Mit authentischem Material wird eine Eichreihe erstellt, anhand derer die Konzentration unbekannter Proben bestimmt werden kann.

### b) chirale Analytik

- stationäre Phase:: Chiracel OD-H, 250*4, 6µm, Daicel, temperiert auf 40°C
- mobilePhase:: Laufmittel A: n-Hexan Laufmittel B: iso-Propanol isokratisch mit 2.5% B
- Flussrate:: 1.0ml/min
- Detektion:: UV-Detektion bei 230 und 260nm
- Retentionszeiten:: 3-Chlor-1-(thien-2-yl)-propan-1-on: ca. 9.5 min (1S)-3-Chlor-1-(thien-2-yl)-propan-1-ol: ca. 16.6 min (1R)-3-Chlor-1-(thien-2-yl)-propan-1-ol: ca. 18.3 min

Mit authentischem Material wird eine Eichreihe erstellt, anhand derer die Konzentration unbekannter Proben bestimmt werden kann.

### Beispiel 2:

### Bereitstellung von Glucose-Dehydrogenase zur Cofaktor-Regenerierung

Zur Kofaktor-Regenerierung kann Glucose-Dehydrogenase verwendet werden. Das Enzym ist aus kommerziellen Quellen (z.B. Jülich Fine Chemicals Order-No. 22.10 oder 19.10) zugänglich oder kann auch leicht basierend auf der bekannten DNA-Sequenz hergestellt werden.
Es wurde ein E. coli XL10 Gold Klon der im Plasmid pUC19 das Glucose-Dehydrogenase-Gen aus Bacillus subtilis (Genbank-Acc.No. M12276) enthält verwendet; dieses Konstrukt trägt die Bezeichnung E. coli LU11293.

Zur Fermentation von E. coli LU11293 wurde folgendes Medium angesetzt:

| | |
|---|---|
| 560 g | Hefeextrakt (65 %) |
| 448 g | Trypton (Difco) |
| 42 g | KH₂PO₄ |
| 84 g | Na₂HPO₄ |
| 644 g | Glycerin (99%) |
| 100 mL | SL4 Lösung (5 fach) |
| | |
| 1 g | Tegosipon 3062 |
| | Medium mit Wasser auf 13,5 L auffüllen, pH-Wert |
| | auf 7,0 einstellen, ca. 300 mL für Vorkultur ent- |
| | nehmen, danach 30 min. bei 122 °C sterilisieren. |
| | |
| | Sterile Salzlösung* zugeben (vorher die Salzlösung für die Schüttelkolben entnehmen, siehe Rapport). |

| | |
|---|---|
| *Salzlösung: 2,1 g CaCl₂ *2 H₂O 3,5 g MgSO₄ * 7 H₂O 14 g NH₄Cl 14 mL Ampicillin-Lösung (100 mg/mL) in 500 mL Wasser lösen und sterilfiltrieren | |

Jeweils 150 mL Medium wurden in zwei 1 L Erlenmeyerkolben sterilisiert und mit 5 mL steriler Salzlösung komplettiert. Nach Beimpfen von einer LB-Ampicillin-Agarplatte wurden die Vorkulturen 12 Stunden bei 37 °C und 200 UPM inkubiert und zum Fermentationsmedium gegeben. Die Fermentation wurde bei 37°C, 0,1 bar Innendruck, pH 7,0 (Regelung mit 20% Phosphorsäure und 25% NaOH) mit einer Begasungsrate von 7,5 Umin und 300 upm gestartet (Regelung pO₂ zwischen 20 und 50% mit 10-20 L/min Zuluft und 500-1500 Upm).. Nach 2 h wurden zur Induktion 0,1 mM IPTG zugegeben und nach insgesamt 13 h die Fermentation beendet. Nach Ernte und Waschen der Zellen (1,3 kg) wurden diese bis zur Verwendung (2-20 g/L im Ansatz) bei -20°C gelagert.

### Beispiel 3:

### Kofaktorregenerierung

Die Regenerierung des Kofaktors kann auch durch die Dehydrogenase, die die Umsetzung von Alkanonen (II) zu Alkanolen (I) bewirkt, durchgeführt werden. In diesem Fall ist die Zugabe eines gesonderten Regenerationsenzyms nicht notwendig. Die Dehydrogenase akzeptiert verschiedene einfache Alkohole als Reduktionsmittel. Sie werden zu den entsprechenden Carbonylverbindungen oxidiert. Ein einfacher Alkohol der sich zur Regeneration von NADH mit Dehydrogenase eignet ist iso-Propanol. Anstelle von Glucosedehydrogenase und Glucose wird 10% iso-Propanol im Reaktionsansatz verwendet.

## Patentansprüche

1. Verfahren zur Herstellung von optisch aktiven-Alkanolen der Formel I 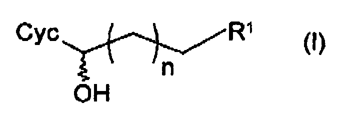 worin
n für einen ganzzahligen Wert von 0 bis 5 steht;
Cyc für einen gegebenenfalls substituierten, ein- oder mehrkernigen, gesättigten oder ungesättigten, carbocyclischen oder heterocyclischen Ring steht, und
R¹ für Halogen, SH, OH, NO₂, NR²R³ oder NR²R³R⁴⁺X⁻ steht, wobei R², R³ und R⁴ unabhängig voneinander für H oder einen Niedrig(C₁-C₈)alkyl- oder Niedrig(C₁-C₈)alkoxy-Rest stehen und X⁻ für ein Gegenion steht,
wobei man in einem Alkanon der Formel II 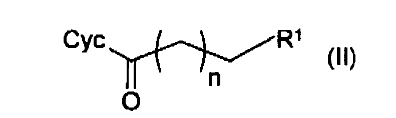 worin n, Cyc und R¹ die oben angegebenen Bedeutungen besitzen,
enthaltenden Medium, ein Enzym mit einer Polypeptidsequenz
(i) SEQ ID NO: 1oder
(ii) bei der bis zu 25% der Aminosäurereste gegenüber SEQ ID NO:1 durch Deletion, Insertion, Substitution oder einer Kombination davon verändert sind und die noch mindestens 50% der enzymatischen Aktivität von SEQ ID NO:1 besitzt, inkubiert,
wobei die Verbindung der Formel II zur Verbindung der Formel I enzymatisch reduziert wird, und man das gebildete Produkt isoliert.

2. Verfahren nach Anspruch 1, zur Herstellung von Derivaten des 1-(2-thienyl)-(S)-propanol der Formel III 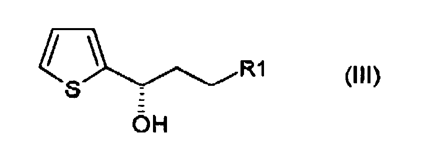 wobei R¹ = Cl oder NHCH₃ bedeuten, wobei man in einem Derivate des 1-(2-thienyl)-propanon der Formel IV 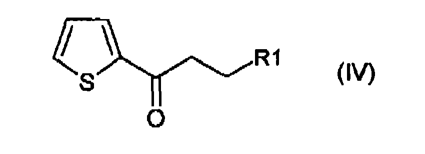 enthaltenden Medium diese Verbindung zur Verbindung der Formel III enzymatisch reduziert wird, und man das enantiomerenreine mit mindestens 90%ee gebildete Produkt isoliert.

3. Verfahren nach einem der vorhergehenden Ansprüche, wobei das Enzym ausgewählt ist unter Enzymen aus Mikroorganismen der Gattung Lactobacillus.

4. Verfahren nach einem der vorhergehenden Ansprüche, wobei man die Umsetzung unter Zugabe von Reduktionsäquivalenten oder die bei der Umsetzung verbrauchten Reduktionsäquivalente regenerierenden Bedingungen durchführt.

5. Verfahren nach Anspruch 4, **dadurch gekennzeichnet, dass** eine Regenierung der Cofaktoren mit einem C₂-C₁₀-Alkanol durchgeführt wird.

6. Verfahren nach einem der vorhergehenden Ansprüche, wobei die Umsetzung der Verbindung der Formel II in Gegenwart eines Mikroorganismus erfolgt, der ausgewählt ist unter Bakterien der Familien Enterobacteriaceae, Pseudomonadaceae, Rhizobiaceae, Streptomycetaceae und Nocardiaceae.

7. Verfahren nach einem der vorhergehenden Ansprüche, wobei der Mikroorganismus ein rekombinanter Mikroorganismus ist, der mit einem Nukleinsäurekonstrukt transformiert ist, welche für ein Enzym gemäß der Definition in Anspruch 1 kodiert.

8. Enzym mit Dehydrogenase-Aktivität mit einer Polypeptidsequenz
(i) SEQ ID NO: 1 oder
(ii) bei der bis zu 25% der Aminosäurereste gegenüber SEQ ID NO:1 durch Deletion, Insertion, Substitution oder einer Kombination davon verändert sind und die noch mindestens 50% der enzymatischen Aktivität von SEQ ID NO:1 besitzt.

9. Verwendung eines Enzyms mit Dehydrogenase-Aktivität mit einer Polypeptidsequenz
(i) SEQ ID NO: 1 oder
(ii) bei der bis zu 25% der Aminosäurereste gegenüber SEQ ID NO:1 durch Deletion, Insertion, Substitution oder einer Kombination davon verändert sind und die noch mindestens 50% der enzymatischen Aktivität von SEQ ID NO:1 besitzt.
zur Herstellung von Verbindungen der Formeln I oder III.

10. Verwendung nach Anspruch 9 in einem Verfahren zur Herstellung von Duloxetine.

## Claims

1. A process for preparing optically active alkanols of the formula I 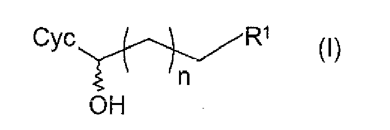 in which
n is an integer from 0 to 5;
Cyc is an optionally substituted, mono- or polynuclear, saturated or unsaturated, carbocyclic or heterocyclic ring, and
R¹ is halogen, SH, OH, NO₂, NR²R³ or NR²R³R⁴⁺X⁻, with R², R³ and R⁴ independently of one another being hydrogen or a lower (C₁-C₆) alkyl or lower (C₁-C₆) alkoxy radical and X⁻ being a counterion,
which process comprises incubating in a medium comprising alkanone of the formula II 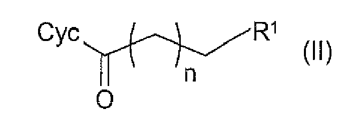 in which n, Cyc and R¹ are as defined above,
an enzyme having a polypeptide sequence
(i) SEQ ID NO: 1 or
(ii) in which, compared to SEQ ID NO:1, up to 25% of the amino acid radicals have been altered by deletion, insertion, substitution or a combination thereof and which retains at least 50% of the enzymic activity of SEQ ID NO:1,
with the compound of the formula II being enzymically reduced to give the compound of the formula I, and isolating the product formed.

2. The process according to claim 1, for preparing derivatives of the 1-(2-thienyl)-(S)-propanol of the formula III 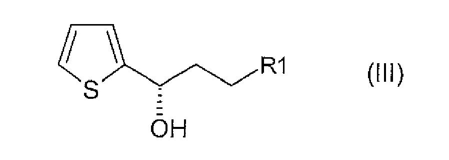 where R¹ = Cl or NHCH₃,
wherein in a medium comprising derivatives of the 1-(2-thienyl)propanone of the formula IV 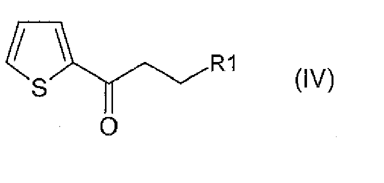 this compound is enzymically reduced to give the compound of the formula III and the enantiomerically pure product formed with at least 90% ee is isolated.

3. The process according to any of the preceding claims, wherein the enzyme is selected from among enzymes of microorganisms of the genus Lactobacillus.

4. The process according to any of the preceding claims, wherein the reaction is carried out with addition of reduction equivalents or under conditions regenerating the reduction equivalents consumed in the reaction.

5. The process according to claim 4, wherein the cofactors are regenerated using a C₂-C₁₀-alkanol.

6. The process according to any of the preceding claims, wherein the compound of the formula II is reacted in the presence of a microorganism selected from among bacteria of the families Enterobacteriaceae, Pseudomonadaceae, Rhizobiaceae, Streptomycetaceae and Nocardiaceae.

7. The process according to any of the preceding claims, wherein the microorganism is a recombinant microorganism which has been transformed with a nucleic acid construct coding for an enzyme according to the definition in claim 1.

8. An enzyme with dehydrogenase activity, having a polypeptide sequence
(i) SEQ ID NO: 1 or
(ii) in which, compared to SEQ ID NO:1, up to 25% of the amino acid radicals have been altered by deletion, insertion, substitution or a combination thereof and which retains at least 50% of the enzymic activity of SEQ ID NO:1.

9. The use of an enzyme with dehydrogenase activity, having a polypeptide sequence
(i) SEQ ID NO: 1 or
(ii) in which, compared to SEQ ID NO:1, up to 25% of the amino acid radicals have been altered by deletion, insertion, substitution or a combination thereof and which retains at least 50% of the enzymic activity of SEQ ID NO:1,
for preparing compounds of the formulae I or III.

10. The use according to claim 9 in a process for preparing duloxetine.

## Revendications

1. Procédé pour la préparation d'alcanols optiquement actifs de formule I 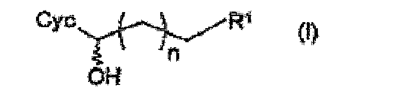 où
n représente une valeur entière de 0 à 5 ;
Cyc représente un cycle carbocyclique ou hétérocyclique le cas échéant substitué, à un ou plusieurs noyaux, saturé ou insaturé, et
R¹ représente halogène, SH, OH, NO₂, NR²R³ ou NR²R³R⁴⁺X⁻, où R², R³ et R⁴ représentent, indépendamment l'un de l'autre, H ou un radical (C₁-C₈)alkyle inférieur ou (C₁-C₈)alcoxy inférieur et X⁻ représente un contre-ion,
où on incube dans un milieu contenant une alcanone de formule II 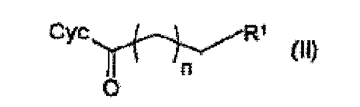 où n, Cyc et R¹ présentent les significations indiquées ci-dessus,
une enzyme présentant une séquence polypeptidique
(i) SEQ ID NO : 1, ou
(ii) dans laquelle jusqu'à 25% des restes d'acides aminés sont modifiés par rapport à la séquence SEQ ID NO : 1 par déplétion, insertion, substitution ou une combinaison de celles-ci et qui présente encore au moins 50% de l'activité enzymatique de la SEQ ID NO : 1,
où le composé de formule II est réduit enzymatiquement en composé de formule I et on isole le produit formé.

2. Procédé selon la revendication 1, pour la préparation de dérivés du 1-(2-thiényl)-(S)-propanol de formule III 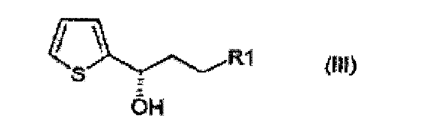 R¹ = Cl ou NHCH₃,
dans lequel on réduit enzymatiquement dans un milieu contenant des dérivés de la 1-(2-thiényl)-propanone de formule IV 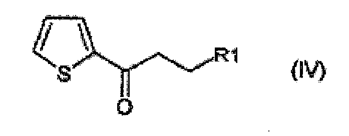 ce composé en composé de formule III, et on isole le produit formé sous une forme énantiomère pure présentant au moins 90% d'ee.

3. Procédé selon l'une quelconque des revendications précédentes, où l'enzyme est choisie parmi les enzymes de microorganismes du genre Lactobacillus.

4. Procédé selon l'une quelconque des revendications précédentes, où on réalise la transformation avec addition d'équivalents de réduction ou dans les conditions de régénération des équivalents de réduction consommés lors de la transformation.

5. Procédé selon la revendication 4, **caractérisé en ce qu'**on réalise une régénération des cofacteurs avec un C₂-C₁₀-alcanol.

6. Procédé selon l'une quelconque des revendications précédentes, où la transformation du composé de formule II est réalisée en présence d'un microorganisme, qui est choisi parmi les bactéries des familles des Enterobacteriaceae, des Pseudomonadaceae, des Rhizobiaceae, des Streptomycetaceae et des Nocardiaceae.

7. Procédé selon l'une quelconque des revendications précédentes, où le microorganisme est un microorganisme recombinant, qui est transformé avec un produit de construction acide nucléique qui code pour une enzyme selon la définition dans la revendication 1.

8. Enzyme avec une activité de déshydrogénase présentant une séquence polypeptidique
(i) SEQ ID NO : 1, ou
(ii) dans laquelle jusqu'à 25% des restes d'acides aminés sont modifiés par rapport à la séquence SEQ ID NO : 1 par délétion, insertion, substitution ou une combinaison de celles-ci et qui présente encore au moins 50% de l'activité enzymatique de la SEQ ID NO : 1.

9. Utilisation d'une enzyme avec une activité de déshydrogénase présentant une séquence polypeptidique
(i) SEQ ID NO : 1, ou
(ii) dans laquelle jusqu'à 25% des restes d'acides aminés sont modifiés par rapport à la séquence SEQ ID NO : 1 par délétion, insertion, substitution ou une combinaison de celles-ci et qui présente encore au moins 50% de l'activité enzymatique de la SEQ ID NO : 1
pour la préparation de composés de formule I ou III.

10. Utilisation selon la revendication 9 dans un procédé pour la préparation de Duloxétine.


### SEQUENCE LISTING

<110> BASF Aktiengesellschaft
<120> Neues Protein zur Synthese von optisch aktiven Alkoholen
<130> PF 57486
<160> 1
<170> PatentIn version 3.1
<210> 1
   <211> 249
   <212> PRT
   <213> Lactobacillus brevis
<400> 1 
Met Thr Asp Arg Leu Lys Asp Lys Val Ala Ile Ile Thr Gly Gly Val
1               5                   10                  15

Ala Gly Ile Gly Leu Gly Ile Ala Glu Cys Tyr Val Arg Glu Gly Ala
            20                  25                  30

Lys Val Val Val Thr Ala Asn His Asn Val Asp Gly Gly His Ala Ala
        35                  40                  45

Val Ala Lys Phe Gly Asp Asp Val Ser Leu Phe Val Gln Gln Asp Val
    50                  55                  60

Ser Lys Glu Ala Asp Trp Gln Lys Val Ile Asp Ala Thr Ile Ala Lys
65                  70                  75                  80

Phe Gly Arg Val Asp Ile Leu Val Asn Asn Ala Gly Ile Gly Gly Val
                85                  90                  95

Asn Thr Ala Ile Glu Asp Leu Asp Leu Ala Asp Trp Gln Lys Val Ile
            100                 105                 110

Asp Val Asn Leu Thr Ala Asn Phe Leu Gly Glu Lys Ala Ala Ile Lys
        115                 120                 125

Ala Met Lys Gln Thr Ala Asp Ala Lys Gly Ser Ile Ile Asn Val Ser
    130                 135                 140
 
Ser Val Ala Gly Leu Val Gly Leu Pro Met Ala Pro Ala Tyr Ser Ala
145                 150                 155                 160

Ser Lys Gly Gly Ser Arg Leu Leu Thr His Ala Thr Ala Leu Asn Leu
                165                 170                 175

Ala Gln Arg Gly Ile Asp Ile Arg Val Asn Ser Val His Pro Gly Trp
            180                 185                 190

Ile Asp Thr Ser Ile Val Pro Glu Ala Ala Arg Gln Gln Ile Ile Ala
        195                 200                 205

Thr Ile Pro Val Gly His Met Gly Gln Pro Gln Asp Ile Gly Glu Ile
    210                 215                 220

Cys Val Tyr Leu Gly Ser Asp Glu Ser Arg Phe Ala Asn Gly Ala Glu
225                 230                 235                 240

Phe Thr Val Asp Gly Gly Gln Arg Ala
                245
PF 57486
   2
PF 57486
   26.10.06 DP/SA